# EUROPEAN PATENT SPECIFICATION

(11) **EP 0 515 484 B1**
(45) Date of publication and mention of the grant of the patent: **26.04.1995**
(21) Application number: 91904432.1
(22) Date of filing: 13.02.1991
(51) Int. Cl.: C07H 21/00, C12N 15/10

(54) **METHOD TO ISOLATE MACROMOLECULES USING MAGNETICALLY ATTRACTABLE BEADS WHICH DO NOT SPECIFICALLY BIND THE MACROMOLECULES**
VERFAHREN ZUR ISOLIERUNG VON MAKROMOLEKÜLEN MITTELS MAGNETISCH ANZIEHBARER KUGELN, DIE DIE MAKROMOLEKÜLE UNSPEZIFISCH BINDEN
PROCEDE D'ISOLATION DE MACROMOLECULES UTILISANT DES SPHERES POUVANT SUBIR UNE ATTRACTION MAGNETIQUE ET NE FORMANT PAS DE LIAISON SPECIFIQUE AVEC LES MACROMOLECULES

(30) Priority: 13.02.1990 GB 9003253
(43) Date of publication of application: 02.12.1992
(73) Proprietor: AMERSHAM INTERNATIONAL plc, Little Chalfont Buckinghamshire HP7 9NA (GB)
(72) Inventor: REEVE, Michael, Alan, Henley-on-Thames RG9 1TE (GB)
(74) Representative: Pennant, Pyers
(86) International application number: GB9100212
(87) International publication number: WO9112079

(56) References cited:
- EP-A- 0 162 819
- EP-A- 0 281 390
- WO-A-40/1503
- BE-A- 686 243
- US-A- 3 470 067
- US-A- 4 001 197
- Chemical Abstracts, vol. 112, no. 11, 12 March 1990, (Columbus, Ohio, US), S. Flygare et al.: see page 606, abstract 96880s, & Enzyme Miccrob. Technol. 1990, 12(2) 95-103
- Chemical Abstracts, vol. 82, no. 18., 5 May 1975, (Columbus Ohio, US), G. Bitton et al.: see page 278, abstract 115953y, & Water Res. 1974, 8 (8), 549-51
- Chemical Abstracts, vol 77, no. 14,2 October 1972, (Columbus Ohio US), J. Warren.: see page 308, abstract 92767w, & Immunization Jap. Encephalitis, Conf. 1969 (Pub. 1971), 152-4
- Chemical Abstracts, vol. 95, no. 9, 31 August 1981, (Columbus, Ohio US), P. A. Munro et al.: see pages 394-395, abstract 76474q, & Biotechnol. Lett. 1981, 3(6), 297-302

## Description

### I Introduction

Many techniques in Molecular Biology, Biochemistry and Chemistry rely upon the process of precipitation. There are two types of precipitation.

In the first type of precipitation, the components from a complex solution that are not of interest are selectively precipitated. The precipitate and supernatant are then separated (usually by centrifugation or filtration) and the supernatant is kept for further use.

In the second type of precipitation, the components of interest from a complex solution are selectively precipitated. The precipitate and supernatant are separated (again by centrifugation or filtration) and the precipitate is kept for further use. This precipitate may well be redissolved for further use.

Examples of precipitation that are of particular relevance to this invention will now be discussed.

### a. Alcohol Precipitation of Nucleic Acid Molecules from Solution:

Alcohol precipitation of nucleic acid molecules from solution is a standard procedure for the concentration and/or purification of these species from complex solutions. Typical methods involve the addition of salt (e.g. 0.1 volumes of 2.5 M sodium acetate (pH 5.2)) to a solution containing nucleic acids followed by addition of an alcohol (e.g. 2.5 volumes of ethanol). The nucleic acids then precipitate. The precipitated nucleic acid molecules aggregate (usually with the aid of reduced temperatures; e.g. 5 minutes on dry ice) and are recovered by centrifugation. After removal of the supernatant, the pelleted precipitate is normally redissolved in the required volume of an appropriate buffer. The nucleic acid may be DNA (partially or wholly single or double stranded), RNA (partially or wholly single or double stranded), mixtures of any of the above or a hybrid RNA/DNA species. The salt used may be sodium acetate, sodium chloride, potassium acetate, potassium chloride, ammonium acetate, ammonium chloride, guanidinium thiocyanate, guanidinium isothiocyanate, guanidinium chloride or mixtures of the above. The alcohol used is normally ethanol or isopropanol.

### b. Precipitation of Bacteriophage and Other Viruses from Solution:

Precipitation of bacteriophage and other viruses from solution by the addition of solutions containing high concentrations of highly hydratable polymers, such as polyethylene glycol (PEG), and salts, such as sodium chloride, is a standard procedure for the concentration and/or purification of these species from complex solutions. The bacteriophage or other viruses precipitated in this way may be used for nucleic acid extraction, protein extraction, infection of host cells, structural studies or immunological studies. A typical procedure involves the addition of 0.2 volumes of 20% (w/v) PEG in 2.5 M sodium chloride to the complex solution known to contain the bacteriophage or other viruses. The bacteriophage or other viruses precipitate. The precipitated particles then aggregate (normally with the aid of incubation at reduced temperatures; e.g. 60 minutes at 4°C) and are recovered by centrifugation. After removal of the supernatant, the pellet (comprising precipitated particles of bacteriophage or other viruses) is normally redissolved in the required volume of an appropriate buffer. The bacteriophage may be filamentous (e.g. M13) or complex (e.g. lambda). They may infect bacteria, animal or plant cells and they may be DNA-containing or RNA-containing.

### c. Removal of Bacterial DNA, Proteins and Membranes from Bacterial Lysates:

Another type of precipitation of interest to Molecular Biologists is used for the removal of bacterial DNA, proteins and membranes from bacterial lysates containing, in addition to the above, RNA and plasmid DNA and/or cosmid DNA and/or bacteriophage DNA. This forms the basis of the alkaline lysis procedure for preparations of low molecular weight DNA. In this procedure, the bacterial cells (e.g. E.coli) are lysed by treatment with sodium hydroxide (e.g. 200 mM) and the detergent sodium dodecyl sulphate (SDS) (e.g. 0.3-1.0% (w/v)). Addition of a mixture of either sodium or potassium acetate at low pH (e.g. 0.5 times the volume of lysis buffer of 3 M sodium or potassium acetate adjusted to pH 4.8 with acetic acid) leads to the formation of a precipitate containing protein, membrane fragments and the entrapped bacterial DNA. The RNA and low molecular weight DNA species are not entrapped in this precipitate and can be recovered from the supernatant after centrifugation or filtration of the precipitate. The low molecular weight DNA species can be purified and/or concentrated, along with cellular RNA, by subsequent alcohol precipitation from this supernatant as described above. The DNA species extracted by this procedure may be plasmid, cosmid or bacteriophage-derived. The volume of cells lysed can be as little as a few microlitres or as large as many litres of bacterial culture.

### Prior Art

EP-A-0162819 describes magnetic spheres comprising magnetic particles enclosed in a polymer matrix, and the use of such spheres in a diagnostic assay in which the spheres are adsorbed on cell walls or incorporated in cells.

US-A-4,001,197 describes a magnetic separation method for recovering single cell protein from single cell organisms, by dispersing magnetic seed in a fluid medium for attachment to the single cell organisms.

US-A-3,470,067 describes the use of particulate magnetic iron oxide to prepare iron oxide-virus vaccine complexes.

EP-A-0281390 describes the use of a polycationic solid support to selectively adsorb nucleotide multimers.

Biotechnology Letters, Vol. 3, No. 6, pages 297-302, 1981, teaches that use of magnetic particles to seed a biological precipitate makes the precipitate amenable to magnetic separation technology. Separation of nucleic acid or protein precipitates during an enzyme isolation process are mentioned as suitable steps for application of the seeding technique.

### II Description of the Invention

In one aspect this invention provides a method of making a nucleic-acid-containing liquid by treating a solution by the use of magnetically attractable beads which do not specifically bind the nucleic acid, comprising the steps of:
- precipitating the nucleic acid out of solution in the presence of the suspended magnetically attractable beads whereby the nucleic acid precipitate becomes non-specifically associated with the beads,
- applying a magnetic field to draw down a precipitate of the nucleic acid and associated beads,
- separating the precipitate from the supernatant liquid,
- adding liquid to the precipitate to re-dissolve the nucleic acid and re-suspend the beads,
- applying a magnetic field to draw down the beads, and
- separating a supernatant liquid containing the nucleic acid from the beads.

The key to the invention is the use of magnetically attractable beads (hereinafter magnetic beads). The nature of the magnetic beads is not critical, and commercially available beads may be used. The beads typically have an average diameter in the range 1 to 100 »m, and comprise finely divided magnetizable material encapsulated in organic polymer.

Or the organic polymer may be omitted. Beads of magnetic iron oxide are commercially available. Such beads have been successfully used in this invention in sizes ranging from below 1 »m up to 40 »m. Even the larger beads remain in suspension at least for the duration of the precipitation step; their subsequent tendency to settle out assists the magnetic field in drawing down the precipitate.

To improve recovery of precipitated biopolymers, the beads may be pretreated to reduce any unwanted tendency to bind the polymers permanently. For example, when the polymers are nucleic acids, the beads may be pre-treated with a phosphate solution. This treatment is believed to phosphatize any exposed magnetisable material, and may not be necessary if the magnetisable material is completely encapsulated in inert polymer.

The beads are preferably added to the solution either before, or together with, a reagent used to effect precipitation. Alternatively, the beads may be added after the precipitation step, under conditions to cause the pre-existing precipitate to become associated with them.

The starting solution is preferably aqueous. However starting solutions in polar or non-polar organic solvents are envisaged.

Biopolymers are polymers found in biological systems. The nature of the biopolymer is not critical to the invention. Biopolymers include nucleic acids (DNA and RNA), proteins, polypeptides, polysaccharides, cell membrane material, bacteriophages, virus, and procaryotic and eucaryotic cells.

At the outset, a biopolymer is present in solution, the term solution being used broadly to cover permanently stable suspensions in which the biopolymer molecules are not aggregated.

It is a feature of the invention that the magnetic beads do not specifically bind the biopolymer. By this feature, the present invention is distinguished from many prior techniques which involve providing a coating on the surface of magnetic beads designed to specifically bind the substance to be drawn down out of solution. When a biopolymer is precipitated out of solution in the presence of the suspended magnetic beads, it becomes non-specifically associated with the beads. When the beads are drawn down by an applied magnetic field, the associated precipitated biopolymer is drawn down with them. But when in solution, the biopolymer does not become associated with the beads.

When the solute is of more interest than the solvent, the method may be used either to concentrate an initially dilute solution, or to recover one or more biopolymers from a mixture of biopolymers, or for both these purposes in sequence. For a sequence of manipulations, the same beads can conveniently be used. The nature of the liquids used to dissolve or re-dissolve the biopolymer, and of reagents used to precipitate biopolymer, are not material to this invention. A skilled reader will have no difficulty in choosing liquids and reagents appropriate to his needs.

In another aspect, the invention provides magnetically attractable beads capable of non-specific association with a nucleic acid, wherein the beads have a coating of phosphate which reduces the tendency of the beads to bind a nucleic acid.

The invention will now be discussed with reference to the three types of precipitation given in the introduction.

### a. Alcohol Precipitation of Nucleic Acid Molecules from Solution:

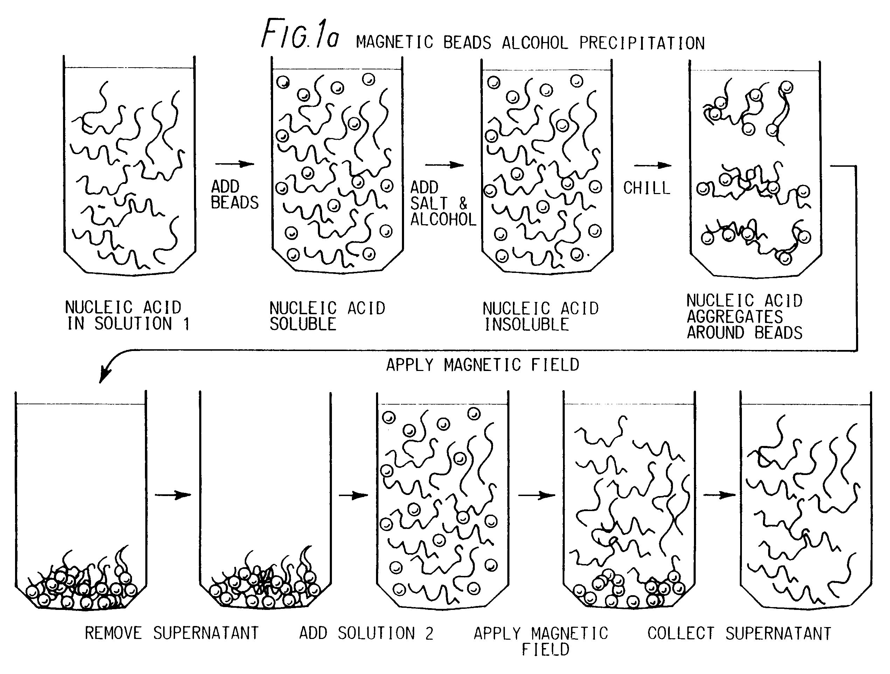

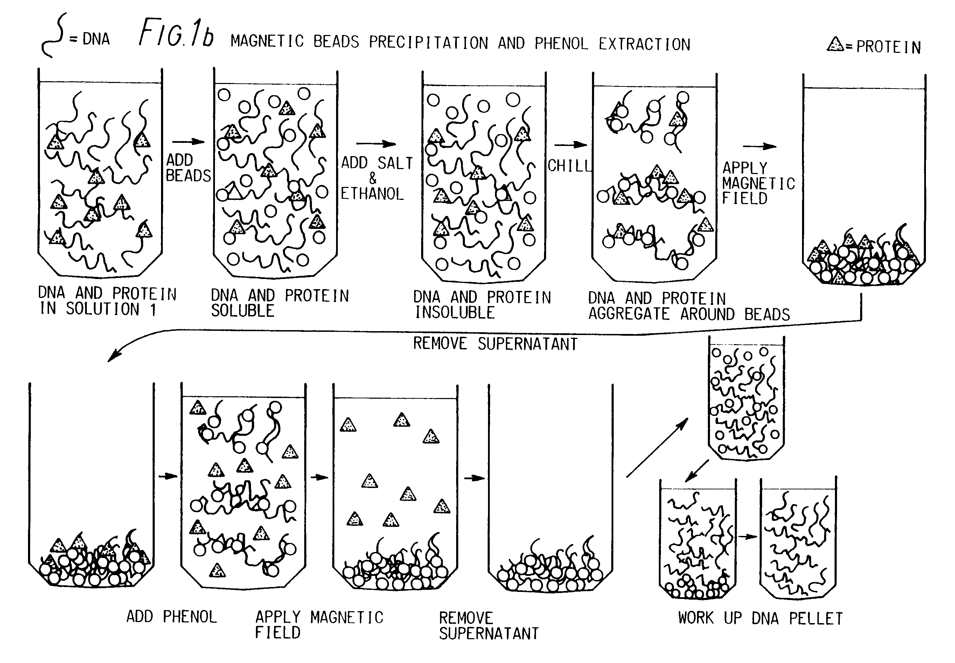

Magnetic bead induced precipitate separation can be used to greatly improve the process of alcohol precipitation of nucleic acids. The alcohol precipitation procedure as modified by this invention is shown in Figure 1a. Magnetic beads are added to the nucleic acid in solution. Salt is then added (the magnetic beads can also be added at the same time as the salt). The nucleic acid is still soluble at this stage. Alcohol is then added. This causes the nucleic acid to come out of solution. The precipitated nucleic acid aggregates around the suspended magnetic beads (which may well act as nucleation sites for this aggregation process). The aggregation stage may be assisted for some types of precipitations by chilling (though chilling does not appear to be necessary for simple precautions of plasmid, phage DNA, RNA and genomic DNA by this method). A magnetic field is then applied to the precipitation. This magnetic field is used to draw the complex of magnetic beads and precipitated nucleic acid to the bottom (or side) of the tube. The supernatant is then removed from the tube. At this point, the precipitate can be washed with ethanol, and/or isopropanol and/or 70% (v/v) ethanol to remove any residual salt, nucleotides, chemicals or organic solvents remaining from treatments of the nucleic acid prior to the precipitation step. The nucleic acid is insoluble in isopropanol, ethanol and 70% (v/v) ethanol. The nucleic acid therefore remains aggregated around the magnetic beads during washing. The washing step can thus be performed vigorously (e.g. by vortex mixing) without risk of losing the precipitate. After the washing step, if performed, the precipitate is redissolved in the required volume of an appropriate buffer in the absence of the magnetic field. Reapplication of the magnetic field to the tube results in just the magnetic beads being drawn to the bottom (or side) of the tube (as the nucleic acid is now dissolved rather than a precipitate as before). The redissolved nucleic acid can now be separated from the magnetic beads by collecting the supernatant containing the dissolved nucleic acid with a pipette whilst the beads are held against the bottom (or side) of the tube by the magnetic field.

The modification of alcohol precipitation by this invention has several clear advantages over the conventional method of precipitation using centrifugation. The procedure, as modified by this invention, is:
1. Faster (the modified procedure takes only 1-2 minutes, as opposed to 10-30 minutes for the conventional procedure using centrifugation).
2. Not reliant upon centrifugation equipment.
3. Readily suited to automation (a great many tubes could be placed over a large electromagnet and these could all be alcohol precipitated simultaneously using a multi channel pipetting device).
4. Especially effective if the precipitate of nucleic acid is to be washed with isopropanol, ethanol or 70% ethanol (e.g. to remove any residual salt, nucleotides or organic solvents such as phenol). Washing can be performed rapidly with no risk of loss of material as can occur with the conventional method based upon centrifugation (where the pellet often detaches from the bottom of the tube during such washing).

Magnetic bead induced precipitate separation can also be used to greatly improve the process of deproteinization and alcohol precipitation of nucleic acids. The deproteinization and alcohol precipitation procedure as modified by this invention is shown in Figure 1b. DNA is given as the example in Figure 4b, though the process is equally applicable to any type of nucleic acid. Magnetic beads are added to the protein and nucleic acid in solution. Salt is then added (the magnetic beads can also be added at the same time as the salt). The protein and nucleic acid are still soluble at this stage. Alcohol is then added. This causes the protein and nucleic acid to come out of solution. The precipitated protein and nucleic acid aggregate around the suspended magnetic beads (which may well act as nucleation sites for this aggregation process). The aggregation stage may be assisted for some types of precipitations by chilling (though chilling does not appear to be necessary for simple precipitations of plasmid, phage DNA, RNA and genomic DNA with protein extraction by this method). A magnetic field is then applied to the precipitation. This magnetic field is used to draw the complex of magnetic beads and precipitated protein and nucleic acid to the bottom (or side) of the tube. The supernatant is then removed from the tube. The protein and nucleic acid remain aggregated around the magnetic beads. Phenol and/or phenol/chloroform and/or phenol/ethanol is then added and the magnetic beads resuspended in the absence of the magnetic field. This resuspension extracts the precipitated protein from the magnetic beads whilst the nucleic acid remains still attached. A magnetic field is again applied to the tube. This magnetic field is used to draw the complex of precipitated nucleic acid and magnetic beads to the bottom (or side) of the tube. The phenolic supernatant (containing the extracted protein) is then removed from the tube. At this point, the precipitate can be washed with ethanol, and/or isopropanol and/or 70% (v/v) ethanol to remove any residual salt, nucleotides, chemicals or organic solvents remaining. The nucleic acid is insoluble in isopropanol, ethanol and 70% (v/v) ethanol. The nucleic acid therefore remains aggregated around the magnetic beads during washing. The washing step can thus be performed vigorously (e.g. by vortex mixing) without risk of losing the precipitate. After the washing step, if performed, the precipitate is redissolved in the required volume of an appropriate buffer in the absence of the magnetic field. Reapplication of the magnetic field to the tube results in just the magnetic beads being drawn to the bottom (or side) of the tube (as the nucleic acid is now dissolved rather than a precipitate as before). The redissolved nucleic acid can now be separated from the magnetic beads by collecting the supernatant containing the dissolved nucleic acid with a pipette whilst the beads are held against the bottom (or side) of the tube by the magnetic field.

The modification of deproteinization and alcohol precipitation by this invention has several clear advantages over the conventional method of using centrifugation. The procedure, as modified by this invention, is:
1. Faster (the modified procedure takes only 5-10 minutes, as opposed to 20-40 minutes for the conventional procedure using centrifugation).
2. Not reliant upon centrifugation equipment.
3. Readily suited to automation (a great many tubes could be placed over a large electromagnet and these could all be deproteinized and alcohol precipitated simultaneously using a multi channel pipetting device).
4. Especially effective if the precipitate of nucleic acid is to be washed with isopropanol, ethanol or 70% ethanol (e.g. to remove any residual salt, nucleotides or organic solvents such as phenol). Washing can be performed rapidly with no risk of loss of material as can occur with the conventional method based upon centrifugation (where the pellet often detaches from the bottom of the tube during such washing).

### b. Precipitation of Bacteriophage and Other Viruses from Solution:

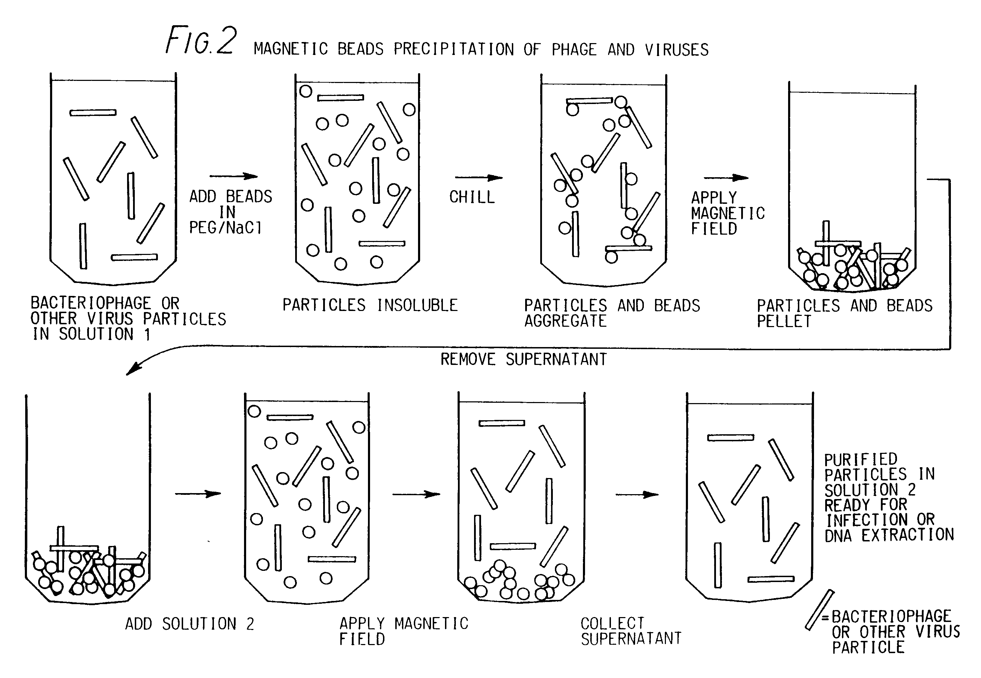

Magnetic bead induced precipitate separation can be used to greatly improve the process of hydrateable polymer/salt precipitation of bacteriophage and other viruses. The hydrateable polymer/salt precipitation procedure as modified by this invention is shown in Figure 2. Magnetic beads, hydrateable polymer (e.g. PEG) and salt (e.g. sodium chloride) are added to the bacteriophage or other viral particles in solution. This causes the particles of bacteriophage or other viruses to come out of solution. The precipitated particles aggregate round the suspended magnetic beads (which may well act as nucleation sites for this aggregation process). The aggregation stage may be assisted for some types of precipitations by chilling (though chilling does not appear to be necessary for simple precipitations of bacteriophage). A magnetic field is then applied to the precipitation. This magnetic field is used to draw the complex of magnetic beads and precipitated particles to the bottom (or side) of the tube. The supernatant is then removed from the tube. The precipitate is redissolved in the required volume of an appropriate buffer in the absence of the magnetic field. Reapplication of the magnetic field to the tube results in just the magnetic beads being drawn to the bottom (or side) of the tube (as the particles of bacteriophage or other viruses are now dissolved rather than a precipitate is before). The redissolved particles of bacteriophage or other viruses can now be separated from the magnetic beads by collecting the supernatant containing the dissolved particles with a pipette whilst the beads are held against the bottom (or side) of the tube by the magnetic field.

The modification of hydrateable polymer/salt precipitation by this invention has several clear advantages over the conventional method of precipitation using centrifugation. The procedure, as modified by this invention, is:
1. Faster (the modified procedure takes only 1-2 minutes, as opposed to 65-75 minutes for the conventional procedure using centrifugation).
2. Not reliant upon centrifugation equipment.
3. Readily suited to automation (a great many tubes could be placed over a large electromagnet and these could all be hydrateable polymer/salt precipitated simultaneously using a multi channel pipetting device).
4. Less likely to produce aerosols of bacteriophage and other viruses than the conventional procedure based upon centrifugation. This is safer if the bacteriophage or other viruses are harmful and will result in less airborne microbial contamination in the laboratory.

### c. Removal of Bacterial DNA, Proteins and Membranes from Bacterial Lysates:

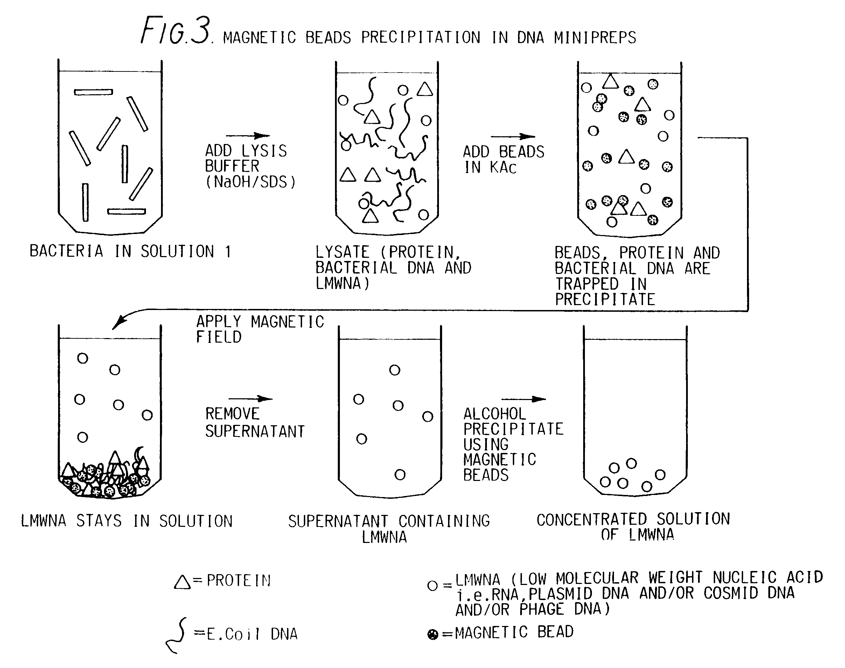

Magnetic bead induced precipitate separation can also be used to greatly improve the precipitation of bacterial DNA, membranes and proteins from bacterial lysates containing RNA and low molecular weight DNA species. The preparation of RNA and low molecular weight DNA species as modified by this invention is shown in Figure 3. Bacteria (containing the low molecular weight DNA species of interest) are lysed with a mixture of sodium hydroxide and SDS. This releases bacterial DNA, proteins, membranes, RNA and low molecular weight DNA into solution. Magnetic beads and either sodium or potassium acetate are then added at low pH. This causes the SDS, proteins and membranes to precipitate. The precipitate also entraps the bacterial DNA and the magnetic beads. A magnetic field is then applied to the precipitation. This magnetic field is used to draw the complex of magnetic beads and precipitated material to the bottom (or side) of the tube. The supernatant is then removed from the tube with a pipette whilst the complex of beads and precipitated material is held against the bottom (or side) of the tube by the magnetic field. The low molecular weight DNA can be purified and/or concentrated from this supernatant (along with any remaining cellular RNA that will also be purified) by alcohol precipitation as described above.

The modification of low molecular weight DNA preparation by this invention has several clear advantages over the conventional method of precipitation using centrifugation. The procedure, as modified by this invention, is:
1. Faster (the modified procedure takes only 5-10 minutes, as opposed to 30-60 minutes for the conventional procedure using centrifugation).
2. Not reliant upon centrifugation equipment.
3. Readily suited to automation (a great many tubes could be placed over a large electromagnet and these could all be precipitated simultaneously using a multi channel pipetting device).

### d. DNA Preparation from Bacteriophage or Other Viruses:

Magnetic bead induced precipitate separation has been shown to greatly improve the process of hydrateable polymer/salt precipitation of bacteriophage and other viruses. The hydrateable polymer/salt precipitation has been shown in Figure 2. Magnetic bead induced precipitate separation has also been shown to greatly improve the precipitation of bacterial DNA, membranes and proteins from bacterial lysates containing RNA and low molecular weight DNA species. The preparation of RNA and low molecular weight DNA species as modified by this invention has been shown in Figure 3. The combination of these two procedures can be used to derive a novel procedure for the purification of low molecular weight DNA from bacteriophage or other viral particles. In this novel procedure, particles of bacteriophage or other viruses are precipitated using the magnetic bead method given in Figure 3. The purified particles are then subjected to lysis by sodium hydroxide and SDS. This step separates the coat proteins from the DNA, with both being released into solution. Magnetic beads and either sodium or potassium acetate are then added at low pH. This causes the SDS and coat proteins to precipitate. The precipitate also entraps the magnetic beads. A magnetic field is then applied to the precipitation. This magnetic field is used to draw the complex of magnetic beads and precipitated material to the bottom (or side) of the tube. The supernatant is then removed from the tube with a pipette whilst the complex of beads and precipitated material is held against the bottom (or side) of the tube by the magnetic field. The low molecular weight DNA can be purified and/or concentrated from this supernatant by alcohol precipitation as described above. The modification of low molecular weight DNA preparation from bacteriophage or other viruses by this invention has several clear advantages over the conventional method of precipitation using centrifugation and other methods. The procedure, as modified by this invention, is:
1. Faster (the modified procedure takes only 5-10 minutes, as opposed to 2-3 hours for the conventional procedure using centrifugation).
2. Not reliant upon centrifugation equipment.
3. Readily suited to automation (a great many tubes could be placed over a large electromagnet and these could all be precipitated simultaneously using a multi channel pipetting device).
4. Not reliant upon organic solvent extraction (e.g. by phenol).
5. Less likely to produce aerosols of bacteriophage and other viruses than the conventional procedure based upon centrifugation. This is safer if the bacteriophage or other viruses are harmful and will result in less airborne microbial contamination in the laboratory.
6. Especially effective if the precipitate of nucleic acid is to be washed with isopropanol, ethanol or 70% ethanol. Washing can be performed rapidly with no risk of loss of material as can occur with the conventional method based upon centrifugation (where the pellet often detaches from the bottom of the tube during such washing).

### e. Precipitation of Bacteria from Solution

Magnetic bead induced precipitate separation can also be used to effect a novel process of alcohol precipitation of cells e.g. bacterial cells. Magnetic beads are added to the bacteria in solution. Salt is then added (the magnetic beads can also be added at the same time as the salt). The bacteria are still soluble at this stage. Alcohol is then added. This causes the bacteria to come out of solution. The precipitated bacteria aggregate around the suspended magnetic beads (which may well act as nucleation sites for this aggregation process). A magnetic field is then applied to the precipitation. This magnetic field is used to draw the complex of magnetic beads and precipitated bacteria to the bottom (or side) of the tube. The supernatant is then removed from the tube. The precipitate is dissolved in the required volume of an appropriate buffer in the absence of the magnetic field. Reapplication of the magnetic field to the tube results in just the magnetic beads being drawn to the bottom (or side) of the tube (as the bacteria are now dissolved rather than a precipitate as before). The redissolved bacteria can now be separated from the magnetic beads by collecting the supernatant containing the dissolved bacteria with a pipette whilst the beads are held against the bottom (or side) of the tube by the magnetic field.

Alternatively the bacteria can be lysed directly on the beads as described in IIIc for DNA preparation. The procedure as effected by this invention is:
1. Faster (the modified procedure takes only 1-2 minutes, as opposed to 5-15 minutes for the conventional procedure using centrifugation).
2. Not reliant upon centrifugation equipment.
3. Readily suited for automation (a great many culture tubes could be placed over a large electromagnet and these could all be alcohol precipitated simultaneously using a multi channel pipetting device).

### III Reduction of the Invention to Practice:

The magnetic beads used were cellulose/ferric oxide (50/50), with a particle size of 1-10 microns diameter. Beads were pretreated by soaking in 100 mM tetrasodium pyrophosphate solution, and stored at 4 degrees in 0.1% (w/v) sodium aside at a concentration of 50 mg/ml.

### a. An Example of Alcohol Precipitation of Nucleic Acid Using Magnetic Bead Induced Precipitate Separation:

### Example 1

Precipitations of plasmid (e.g. pBR322) can be performed according to the following protocol: Take pBR322 DNA in, for example, 100 »l of TE buffer (10 mM Tris-HCl (pH 8.0), 1 mM EDTA (pH 8.0)); add 1/10th volume (i.e. 10 »l) of a solution containing magnetic beads at 50 mg/ml and tRNA at 1 mg/ml (as carrier, this may be omitted if the concentration of nucleic acid is greater than about 10-25 »g/ml) in 2.5 M sodium acetate adjusted to pH 5.2 with acetic acid; mix; add 2.5 volumes (i.e. 250 »l) of ethanol; mix; place over a permanent magnet to bring down the precipitate; remove the supernatant; wash the pellet in, for example, 100 »l of 70% (v/v) ethanol by resuspending with a pipette in the absence of the magnetic field, finally removing the supernatant (wash solution) with a pipette in the presence of the magnetic field; redissolve the pellet in the required volume of TE buffer.

No loss occurs on omission of the chilling step for pBR322 DNA. Also, no loss occurs from washing the precipitate with 70% (v/v) ethanol for pBR322 DNA. The above procedure works equally well for human genomic DNA and for RNA.

### Example 2

Precipitation of pBR322 plasmid DNA with deproteinization can be performed according to the following protocol: Take, for example, pBR322 DNA in 20 »l of TE buffer (10 mM Tris-HCl (pH 8.0), 1 mM EDTA (pH 8.0)) containing protein (e.g. a 1/4 dilution of Rainbow Markers™ (Amersham International)); add 1/10th volume (i.e. 2 »l) of a solution containing magnetic beads at 50 mg/ml and tRNA at 1 mg/ml (as carrier, this may be omitted if the concentration of nucleic acid is greater than about 10-25 »g/ml) in 2.5 M sodium acetate adjusted to pH 5.2 with acetic acid; mix; add 2.5 volumes (i.e. 50 »l) of ethanol; mix; place over a permanent magnet to bring down the precipitate; remove the supernatant; wash the pellet in, for example, 50 »l of phenol (or greater than 60% (v/v) phenol in ethanol) by resuspending with a pipette in the absence of the magnetic field, finally removing the supernatant (wash solution) with a pipette in the presence of the magnetic field; redissolve the pellet in the required volume of TE buffer. The yield of DNA falls off with less than 40% (v/v) phenol in ethanol used for protein extraction. No losses are incurred with this additional step of protein extraction compared to a protein-free ethanol precipitation. Ethanol precipitation from a solution heavily contaminated with protein is also seen to be dependent upon the extraction of the contaminating protein by a phenol containing solution (i.e. the DNA cannot be redissolved from the beads if protein extraction has not been performed). The successful extraction of the protein into the phenolic layer by this procedure can clearly be seen when using coloured proteins. The above procedure works equally well for human genomic DNA and for RNA.

### b. An Example of Hydrateable Polymer/Salt Precipitation of Bacteriophage Using Magnetic Bead Induced Precipitate Separation:

### Example 3

Precipitations can be performed on, for example, 1 ml samples of M13mp8 bacteriophage in 2xTY broth (precleared of bacteria by centrifugation) according to the following protocol: Add 0.4 volumes (i.e. 400 »l) of 2 mg/ml magnetic beads in 20% (w/v) PEG, 2.5 M NaCl; mix; bring down magnetic beads and precipitate using a permanent magnet; redissolve magnetic bead pellet in the required volume of TE buffer.

At 0.4 volumes, the amount of bacteriophage not brought down by the beads is negligible.

### Example 4

DNA preparations can be performed on, for example, 1 ml samples of M13mp8 bacteriophage in 2xTY broth (precleared of bacteria by centrifugation) according to the following protocol: Add 0.4 volumes (i.e. 400 »l) of 2 mg/ml magnetic beads in 20% (w/v) PEG, 2.5 M NaCl; mix; bring down magnetic beads and precipitate using a permanent magnet; redissolve magnetic bead pellet in 1/5th volume (i.e. 200 »l) of TE buffer; extract with an equal volume (i.e. 200 »l) of phenol; remove aqueous (top) layer; add 1/10th volume (i.e. 20 »l) of a solution containing magnetic beads at 50 mg/ml and tRNA at 1 mg/ml (as carrier, this may be omitted if the concentration of nucleic acid is greater than about 10-25 »g/ml) in 2.5 M sodium acetate adjusted to pH 5.2 with acetic acid; mix; add 2.5 volumes (i.e. 500 »l) of ethanol; mix; place over a permanent magnet to bring down the precipitate; remove the supernatant; wash the pellet in, for example, 100 »l of 70% (v/v) ethanol by resuspending with a pipette in the absence of the magnetic field, finally removing the supernatant (wash solution) with a pipette in the presence of the magnetic field; redissolve DNA in the required volume of TE buffer.

### Example 5

DNA preparations can also be performed on, for example, 1 ml samples of M13mp8 bacteriophage in 2xTY broth (precleared of bacteria by centrifugation) according to the following protocol: Add 0.4 volumes (i.e. 400 »l) of 2 mg/ml magnetic beads in 20% (w/v) PEG, 2.5 M NaCl; mix; bring down magnetic beads and precipitate using a permanent magnet; redissolve magnetic bead pellet in 1/10th original volume (i.e. 100 »l) of 4 M sodium perchlorate in TE buffer; now add 2.5 volumes (i.e. 250 »l) of ethanol; mix; place over a permanent magnet to bring down the precipitate; remove the supernatant; wash the pellet twice in, for example, 100 »l of 70% (v/v) ethanol by resuspending with a pipette in the absence of the magnetic field, finally removing the supernatant (wash solution) with a pipette in the presence of the magnetic field; redissolve DNA in the required volume of TE buffer.

### c. An Example of the Removal of Bacterial DNA, Proteins and Membranes from Bacterial Lysates Using Magnetic Bead Induced Precipitate Separation:

### Example 6

pUC19 DNA can be extracted from E.coli MC1061 cells by the following protocol: Take, for example, 250 »l of bacterial culture; add 1/5th volume (i.e. 50 »l) of 1.2 M NaOH, 1.2% (w/v) SDS; mix; incubate 2 minutes at room temperature; now add 3/5th volume (i.e. 150 »l) of 10 mg/ml magnetic beads in 3 M potassium acetate adjusted to pH 4.8 with acetic acid; mix; bring down precipitated material with a permanent magnet and keep supernatant; isopropanol precipitate the supernatant as follows: add 1/10th supernatant volume (i.e. 45 »l) of a solution containing magnetic beads at 50 mg/ml and tRNA at 1 mg/ml (as carrier, this may be omitted if the concentration of nucleic acid is greater than about 10-25 »g/ml) in 2.5 M sodium acetate adjusted to pH 5.2 with acetic acid; mix; add 0.6 supernatant volumes (i.e. 270 »l) of isopropanol; mix; place over a permanent magnet to bring down the precipitate; remove the supernatant; wash the pellet in, for example, 100 »l of 70% (v/v) ethanol by resuspending with a pipette in the absence of the magnetic field, finally removing the supernatant (wash solution) with a pipette in the presence of the magnetic field; redissolve DNA in the required volume of TE buffer. Preparations can be incubated with 10 »g/ml ribonuclease A for 10 minutes at 37°C before analysis.

### Example 7

pUC19 DNA can also be extracted from E.coli MC1061 cells by the following protocol: Take, for example, 500 »l of bacterial culture; add 1 volume (i.e. 500 »l) of a solution containing magnetic beads at 5 mg/ml in 0.2 M sodium acetate (adjusted to pH 5.2 with acetic acid) dissolved in ethanol; mix; bring down precipitated bacteria with a permanent magnet; remove supernatant and discard; redissolve bacterial pellet in, for example, 300 »l of 0.2 M NaOH, 0.2% (w/v) SDS; mix; incubate 2 minutes at room temperature; now add 1/2 volume (i.e. 150 »l) of 3 M potassium acetate adjusted to pH 4.8 with acetic acid; mix; bring down precipitated material with a permanent magnet and keep supernatant; isopropanol precipitate the supernatant as follows: add 1/10th supernatant volume (i.e. 45 »l) of a solution containing magnetic beads at 50 mg/ml and tRNA at 1 mg/ml (as carrier, this may be omitted if the concentration of nucleic acid is greater than about 10-25 »g/ml) in 2.5 M sodium acetate adjusted to pH 5.2 with acetic acid; mix; add 0.6 supernatant volumes (i.e. 270 »l) of isopropanol; mix; place over a permanent magnet to bring down the precipitate; remove the supernatant; wash the pellet in, for example, 100 »l of 70% (v/v) ethanol by resuspending with a pipette in the absence of the magnetic field, finally removing the supernatant (wash solution) with a pipette in the presence of the magnetic field; redissolve DNA in the required volume of TE buffer. Preparations can be incubated with 10 »g/ml ribonuclease A for 10 minutes at 37°C before analysis.

### d. An Example of DNA Extraction from Bacteriophage Using Magnetic Bead Induced Precipitate Separation for: Hydrateable Polymer/Salt Precipitation, Removal of Coat Proteins and Alcohol Precipitation of the DNA:

### Example 8

M13mp8 phage can be precipitated with magnetic beads, PEG and NaCl as described in IIIb. DNA can then be prepared by the alkaline lysis procedure as described in IIIc (dissolving the PEG/NaCl/magnetic beads precipitate of bacteriophage particles in, for example, 250 »l of TE buffer for alkaline lysis). The alkaline lysis method gives M13mp8 DNA at about half the yield of the phenol extraction preparation.

### IV Other Types of Precipitation:

These include the following:
Precipitations of bacteria, tissue culture cells and blood cells by suitable precipitants (e.g. an equal volume of ethanolic 0.2 M sodium acetate adjusted to pH 5.2 with acetic acid for E.coli) and magnetic bead induced precipitate separation.

Ammonium sulphate precipitation of proteins with magnetic bead induced precipitate separation.

Precipitation of proteins by salts other than ammonium sulphate and magnetic bead induced precipitate separation (e.g. sodium perchlorate sodium iodide, guanidinium chloride, guanidinium thiocyanate, guanidinium isothiocyanate and other chaotropic agents).

Precipitation of proteins by denaturants and magnetic bead induced precipitate separation.

Precipitation of proteins by detergents and magnetic bead induced precipitate separation.

Precipitation of nucleic acids by the detergent cetyl trimethyl ammonium bromide and magnetic bead induced precipitate separation.

Precipitation or proteins and/or nucleic acids with agents such as trichloroacetic acid (that denature due to extremes of pH) and magnetic bead induced precipitate separation.

Selective RNA precipitations from lithium chloride and magnetic bead induced precipitate separation.

Selective precipitations of nucleic acids from other nucleic acids (e.g. precipitations of high molecular weight DNA from oligodeoxyribonucleotides and/or deoxynucleotide polyphosphates) which may work better using magnetic bead induced precipitate separation than centrifugation.

Immune precipitations and magnetic bead induced precipitate separation.

Complement fixation precipitations and magnetic bead induced precipitate separation.

Blood clotting precipitations and magnetic bead induced precipitate separation.

Latex bead precipitation assays and magnetic bead induced precipitate separation.

Haemagluttination assays and magnetic bead induced precipitate separation.

## Claims

1. A method of making a nucleic-acid-containing liquid by treating a solution by the use of magnetically attractable beads which do not specifically bind the nucleic acid, comprising the steps of:
- precipitating the nucleic acid out of solution in the presence of the suspended magnetically attractable beads whereby the nucleic acid precipitate becomes non-specifically associated with the beads,
- applying a magnetic field to draw down a precipitate of the nucleic acid and associated beads,
- separating the precipitate from the supernatant liquid,
- adding liquid to the precipitate to re-dissolve the nucleic acid and re-suspend the beads,
- applying a magnetic field to draw down the beads, and
- separating a supernatant liquid containing the nucleic acid from the beads.

2. A method as claimed in claim 1, wherein said solution has been obtained from a starting bacterial lysate by the steps of:
- forming in the bacterial lysate a precipitate selected from the group consisting of cell debris, proteins and chromosomal DNA, in the presence of the suspended magnetically attractable beads which precipitate becomes non-specifically associated with the beads,
- applying a magnetic field to draw down the precipitate and the associated beads, and
- recovering the said solution as a supernatant liquid.

3. A method of making a nucleic-acid-containing liquid by treating a solution comprising nucleic acid and protein by the use of magnetically attractable beads which do not specifically bind the nucleic acid or the protein, comprising the steps of:
- forming a precipitate comprising protein and nucleic acid in the presence of the suspended magnetically attractable particles which precipitate becomes non-specifically associated with the beads,
- applying a magnetic field to draw down the beads and the associated precipitate,
- separating the precipitate from a supernatant liquid ,
- adding liquid to the precipitate to selectively re-dissolve the protein and re-suspend the beads and the associated nucleic acid,
- applying a magnetic field to draw down a precipitate of the nucleic acid and the associated beads,
- separating a supernatant liquid containing the protein from the precipitate,
- adding liquid to the precipitate to redissolve the nucleic acid and re-suspend the beads,
- applying a magnetic field to draw down the beads, and
- separating a supernatant liquid containing the nucleic acid from the beads.

4. A method for recovering low molecular weight nucleic acids from a starting solution of a material selected from the group consisting of bacteriophage, virus, cell and mixtures thereof, by the use of magnetically attractable beads which do not specifically bind the said material, which method comprises the steps:
- precipitating the said material out of solution in the presence of the suspended magnetically attractable beads whereby the material becomes non-specifically associated with the beads,
- applying a magnetic field to draw down a precipitate of the material and the associated beads,
- lysing the said material to form a cell lysate solution comprising protein, membrane, bacterial DNA and low molecular weight nucleic acids,
- precipitating out of solution the protein, membrane and bacterial chromosomal DNA whereby the precipitate becomes non-specifically associated with the beads,
- and recovering the supernatant liquid containing the low molecular weight nucleic acids.

5. A method for recovering low molecular weight nucleic acids from a starting solution of a material selected from the group consisting of bacteriophage, virus, cell and mixtures thereof, by the use of magnetically attractable beads which do not specifically bind the said material, which method comprises the steps:
- precipitating the said material out of solution in the presence of the suspended magnetically attractable beads whereby the material becomes non-specifically associated with the beads,
- applying a magnetic field to draw down a precipitate of the material and the associate beads,
- lysing the said material to form a lysate solution comprising protein and nucleic acids,
- precipitating out of the solution the nucleic acid in the presence of suspended magnetically attractable beads whereby the nucleic acid precipitate becomes non-specifically associated with the beads,
- applying a magnetic field to draw down a precipitate of the nucleic acid and associated beads,
- separating the precipitate from the supernatant liquid,
- adding liquid to the precipitate to re-dissolve the nucleic acid and re-suspend the beads,
- applying a magnetic field to draw down the beads, and
- separating the supernatant liquid containing the nucleic acid from the beads.

6. A method as claimed in any one of claims 1 to 5, wherein the beads have been pre-treated with a phosphate solution.

7. A method as claimed in any one of claims 1 to 6, wherein the solution is in an aqueous medium.

8. Magnetically attractable beads capable of non-specific association with a nucleic acid, wherein the beads have a coating of phosphate which reduces the tendency of the beads to bind a nucleic acid.

9. Magnetically attractable beads as claimed in claim 8, wherein the coating of phosphate has been formed by pre-treating the beads with a phosphate solution.

## Patentansprüche

1. Ein Verfahren zur Herstellung nucleinsäurehaltiger Flüssigkeiten, bei dem eine Lösung mit magnetisch anziehbaren Kugeln behandelt wird, die die Nucleinsäure unspezifisch binden, und das die folgenden Arbeitsschritte umfaßt:
- Fällen der Nucleinsäure aus einer Lösung in Gegenwart schwebender, magnetisch anziehbarer Kugeln, wobei der Nucleinsäure-Niederschlag unspezifisch an die Kugeln gebunden wird,
- Anlegen eines magnetischen Feldes, um die Kugeln und den an sie gebundenen Nucleinsäure-Niederschlag nach unten zu ziehen,
- Trennen des Niederschlags von der Überstandsflüssigkeit,
- Versetzen des Niederschlags mit einer Flüssigkeit, um die Nucleinsäure zu lösen und die Kugeln erneut in den Schwebezustand zu versetzen,
- Anlegen eines magnetischen Feldes, um die Kugeln nach unten zu ziehen und
- Trennen der nucleinsäurehaltigen Überstandsflüssigkeit von den Kugeln.

2. Ein Verfahren wie unter (1) beschrieben, bei dem die genannte Lösung aus einem bakteriellen Ausgangslysat durch die folgenden Schritte gewonnen wurde:
- Bilden eines Niederschlags im bakteriellen Lysat aus der aus Zelltrümmern, Proteinen und chromosomaler DNA bestehenden Gruppe in Gegenwart schwebender, magnetisch anziehbarer Kugeln, an die der Niederschlag unspezifisch gebunden wird,
- Anlegen eines magnetischen Feldes, um die Kugeln und den an sie gebundenen Nucleinsäure-Niederschlag nach unten zu ziehen,
- Rückgewinnen der genannten Lösung als Überstandsflüssigkeit.

3. Ein Verfahren zur Herstellung nucleinsäurehaltiger Flüssigkeiten, bei dem eine aus Nucleinsäure und Protein bestehende Lösung mit magnetisch anziehbaren Kugeln behandelt wird, die die Nucleinsäure oder das Protein unspezifisch binden, und das die folgenden Arbeitsschritte umfaßt:
- Bilden eines Niederschlags aus Protein und Nucleinsäure in Gegenwart schwebender, magnetisch anziehbarer Kugeln, an die der Niederschlag unspezifisch gebunden wird,
- Anlegen eines magnetischen Feldes, um die Kugeln und den an sie gebundenen Nucleinsäure-Niederschlag nach unten zu ziehen,
- Trennen des Niederschlags von der Überstandsflüssigkeit,
- Versetzen des Niederschlags mit einer Flüssigkeit, um das Protein selektiv zu lösen und die Kugeln sowie die an sie gebundene Nucleinsäure wieder in den Schwebezustand zurückzuversetzen,
- Anlegen eines magnetischen Feldes, um den Nucleinsäure-Niederschlag und die an ihn gebundenen Kugeln nach unten zu ziehen,
- Trennen der proteinhaltigen Überstandsflüssigkeit vom Niederschlag,
- Versetzen des Niederschlags mit einer Flüssigkeit, um die Nucleinsäure zu lösen und die Kugeln in den Schwebezustand zurückzuversetzen,
- Anlegen eines magnetischen Feldes, um die Kugeln nach unten zu ziehen und
- Trennen der nucleinsäurehaltigen Überstandsflüssigkeit von den Kugeln.

4. Ein Verfahren zur Rückgewinnung von niedermolekularen Nucleinsäuren aus einer Ausgangslösung, die Bakteriophagen, Viren, Zellen oder Mischungen dieser Materialien enthält, unter Verwendung magnetisch anziehbarer Kugeln, die das genannte Material unspezifisch binden; dieses Verfahren umfaßt die folgenden Arbeitsschritte:
- Fällen des genannten Materials aus der Lösung in Gegenwart schwebender, magnetisch anziehbarer Kugeln, wobei das Material unspezifisch an diese Kugeln gebunden wird,
- Anlegen eines magnetischen Feldes, um die Kugeln und den an sie gebundenen Niederschlag des Materials nach unten zu ziehen,
- Lysieren des genannten Materials, um eine Zellysatlösung zu bilden, die aus Protein, Membranen, bakterieller DNA und niedermolekularer Nucleinsäure besteht,
- Ausfällen des Proteins, der Membranen und der bakteriellen chromosomalen DNA aus der Lösung, wobei der Niederschlag unspezifisch an die Kugeln gebunden wird, und
- Rückgewinnen der Überstandsflüssigkeit, die die niedermolekularen Nucleinsäuren enthält.

5. Ein Verfahren zur Rückgewinnung von niedermolekularen Nucleinsäuren aus einer Ausgangslösung, die Bakteriophagen, Viren, Zellen oder Mischungen dieser Materialien enthält, unter Verwendung magnetisch anziebbarer Kugeln, die das genannte Material unspezifisch binden; dieses Verfahren umfaßt die folgenden Arbeitsschritte:
- Fällen des genannten Materials aus der Lösung in Gegenwart schwebender, magnetisch anziehbarer Kugeln, wobei das Material unspezifisch an diese Kugeln gebunden wird,
- Anlegen eines magnetischen Feldes, um die Kugeln und den an sie gebundenen Niederschlag des Materials nach unten zu ziehen,
- Lysieren des genannten Materials, um eine Lysatlösung zu bilden, die aus Protein und Nucleinsäuren besteht,
- Ausfällen der Nucleinsäure aus der Lösung in Gegenwart schwebender, magnetisch anziehbarer Kugeln, wobei der Nucleinsäure-Niederschlag unspezifisch an die Kugeln gebunden wird,
- Anlegen eines magnetischen Feldes, um die Kugeln und den an sie gebundenen Nucleinsäure-Niederschlag nach unten zu ziehen,
- Trennen des Niederschlags von der Überstandsflüssigkeit,
- Versetzen des Niederschlags mit einer Flüssigkeit, um die Nucleinsäure zu lösen und die Kugeln in den Schwebezustand zurückzuversetzen,
- Anlegen eines magnetischen Feldes, um die Kugeln nach unten zu ziehen, und
- Trennen der nucleinsäurehaltigen Überstandsflüssigkeit von den Kugeln.

6. Ein Verfahren, das den in den Punkten 1 bis 5 beschriebenen Verfahren entspricht, wobei die Kugeln mit einer Phosphatlösung vorbehandelt wurden.

7. Ein Verfahren, das den in den Punkten 1 bis 5 genannten Verfahren entspricht, wobei die Lösung in einem wäßrigen Medium vorliegt.

8. Magnetisch anziehbare Kugeln, die in der Lage sind, eine Nucleinsäure unspezifisch zu binden, sofern die Kugeln einen Phosphatüberzug haben, durch den deren Tendenz, eine Verbindung mit der Nucleinsäure einzugehen, reduziert wird.

9. Magnetisch anziehbare Kugeln, die denen in Punkt 8 genannten entsprechen, wobei der Phosphatüberzug durch Vorbehandlung der Kugeln mit einer Phosphatlösung gebildet worden ist.

## Revendications

1. Une méthode de réalisation d'un liquide contenant de l'acide nucléique en traitant une solution par l'utilisation de perles pouvant subir une attraction magnétique et ne formant pas de liaison spécifique avec l'acide nucléique, comprenant les étapes:
- de la précipitation de l'acide nucléique en solution en présence des perles pouvant subir une attraction magnétique en suspension par quoi le précipité d'acide nucléique devient non-spécifiquement associé aux perles,
- de l'application d'un champ magnétique pour faire descendre un précipité de l'acide nucléique et des perles associées,
- de la séparation du précipité du liquide surnageant,
- de l'ajout de liquide au précipité pour redissoudre l'acide nucléique et re-mettre les perles en suspension.
- de l'application d'un champ magnétique pour faire descendre les perles, et
- de la séparation d'un liquide surnageant contenant l'acide nucléique des perles.

2. Une méthode comme revendiqué dans la revendication 1, dans quoi ladite solution a été obtenue à partir d'un lysat bactérien de départ par les étapes:
- de la formation dans le lysat bactérien d'un précipité sélectionné à partir du groupe composé de débris de cellules, de protéines et d'ADN chromosomique, en présence des perles pouvant subir une attraction magnétique en suspension, ce précipité devenant non-spécifiquement associé aux perles,
- de l'application d'un champ magnétique pour faire descendre le précipité et les perles associées, et
- de la récupération de ladite solution en tant que liquide surnageant.

3. Une méthode de réalisation d'un liquide contenant de l'acide nucléique en traitant une solution comprenant de l'acide nucléique et de la protéine par l'utilisation de perles pouvant subir une attraction magnétique et ne formant pas de liaison spécifique avec l'acide nucléique ou la protéine, comprenant les étapes:
- de la formation d'un précipité comprenant de la protéine et de l'acide nucléique en présence des particules pouvant subir une attraction magnétique en suspension, ce précipité devenant non-spécifiquement associé aux perles,
- de l'application d'un champ magnétique pour faire descendre les perles et le précipité associé,
- de la séparation du précipité d'un liquide surnageant,
- de l'ajout de liquide au précipité pour redissoudre sélectivement la protéine et remettre les perles et l'acide nucléique associé en suspension,
- de l'application d'un champ magnétique pour faire descendre un précipité de l'acide nucléique et les perles associées,
- de la séparation d'un liquide surnageant contenant la protéine du précipité.
- de l'ajout de liquide au précipité pour redissoudre l'acide nucléique et remettre les perles en suspension,
- de l'application d'un champ magnétique pour faire descendre les perles, et
- de la séparation d'un liquide surnageant contenant l'acide nucléique des perles.

4. Une méthode pour la récupération des acides nucléiques de faible poids moléculaire à partir d'une solution de départ d'un matériau sélectionné à partir du groupe composé de bactériophages, de virus, de cellules et de mélanges de ces derniers, par l'utilisation de perles pouvant subir une attraction magnétique et ne formant pas de liaison spécifique avec ledit matériau, cette méthode comprenant les étapes:
- de la précipitation dudit matériau en solution en présence des perles pouvant subir une attraction magnétique en suspension par quoi le matériau devient non-spécifiquement associé aux perles,
- de l'application d'un champ magnétique pour faire descendre un précipité du matériau et les perles associées,
- de la lyse dudit matériau pour former une solution de lysat de cellule comprenant de la protéine, de la membrane, de l'ADN bactérien et des acides nucléiques de faible poids moléculaire,
- de la précipitation en solution de la protéine, de la membrane et de l'ADN chromosomique bactérien par quoi le précipité devient non-spécifiquement associé aux perles,
- et de la récupération du liquide surnageant contenant les acides nucléiques de faible poids moléculaire.

5. Une méthode pour la récupération des acides nucléiques de faible poids moléculaire à partir d'une solution de départ d'un matériau sélectionné à partir du groupe composé de bactériophages, de virus, de cellules et de mélanges de ces derniers, par l'utilisation de perles pouvant subir une attraction magnétique et ne formant pas de liaison spécifique avec ledit matériau, cette méthode comprenant les étapes:
- de la précipitation dudit matériau en solution en présence des perles pouvant subir une attraction magnétique en suspension par quoi le matériau devient non-spécifiquement associé aux perles,
- de l'application d'un champ magnétique pour faire descendre un précipité du matériau et les perles associées,
- de la lyse dudit matériau pour former une solution de lysat comprenant de la protéine et des acides nucléiques,
- de la précipitation en solution de l'acide nucléique en présence des perles pouvant subir une attraction magnétique en suspension par quoi le précipité d'acide nucléique devient non-spécifiquement associé aux perles,
- de l'application d'un champ magnétique pour faire descendre un précipité de l'acide nucléique et les perles associées.
- de la séparation du précipité du liquide surnageant,
- de l'ajout de liquide au précipité pour redissoudre l'acide nucléique et remettre les perles en suspension.
- de l'application d'un champ magnétique pour faire descendre les perles, et
- de la séparation du liquide surnageant contenant l'acide nucléique des perles.

6. Une méthode comme revendiqué dans l'une quelconque des revendications 1 à 5, dans laquelle les perles ont été pré-traitées à l'aide d'une solution de phosphate.

7. Une méthode comme revendiqué dans l'une quelconque des revendications 1 à 6, dans laquelle la solution est dans un milieu aqueux.

8. Des perles pouvant subir une attraction magnétique et pouvant former une association non spécifique avec un acide nucléique, dans quoi les perles ont un revêtement de phosphate qui réduit la tendance des perles à former une liaison avec un acide nucléique.

9. Des perles pouvant subir une attraction magnétique comme revendiqué dans la revendication 8, dans quoi le revêtement de phosphate a été formé en pré-traitant les perles à l'aide d'une solution de phosphate.
